# EUROPÄISCHE PATENTANMELDUNG

(11) **EP 4 624 038 A1**
(43) Veröffentlichungstag der Anmeldung: **01.10.2025**
(21) Anmeldenummer: 24167625.3
(22) Anmeldetag: 28.03.2024
(51) Int. Cl.: B01J 8/06, F28D 7/16, C07C 29/15

(54) **STÜTZELEMENTE MIT STÜTZPLATTEN IN EINEM REAKTORBEHÄLTER**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Oelmann, Tobias, 60439 Frankfurt (DE); Strozyk, Michael, 60439 Frankfurt (DE); Bartels, Katja, 60439 Frankfurt (DE); Tercan, Abdullah Enes, 60439 Frankfurt (DE); Gronemann, Veronika, 60439 Frankfurt (DE)
(74) Vertreter: Air Liquide

(57) **Zusammenfassung**

Vorrichtung (1.1;1.2) umfassend einen Reaktorbehälter (2), ein Rohrbündel (3) mit mehreren Rohren (4), eine erste Stützplatte (5) und eine zweite Stützplatte (6), wobei das Rohrbündel (3) im Reaktorbehälter (2) angeordnet ist, wobei das Rohrbündel (3) eine erste Rohrgruppe (7), eine zweite Rohrgruppe (8) und eine dritte Rohrgruppe (9) umfasst, wobei die erste Stützplatte (5) und die zweite Stützplatte (6) quer zu einer Längsachse (10) des Reaktorbehälters (2) im Reaktorbehälter (2) angeordnet sind, wobei jedes der Rohre (4) durch die erste Stützplatte (5) und die zweite Stützplatte (6) hindurchgeführt ist.

## Beschreibung

Die Erfindung betrifft eine Anordnung zur Methanolsynthese sowie eine Vorrichtung, welche Teil einer solchen Anordnung sein kann. Weiterhin betrifft die Erfindung eine Verwendung der Vorrichtung und ein Verfahren zur Methanolsynthese mit der Vorrichtung.

Bekannt sind Verfahren zur industriellen Herstellung von Methanol durch heterogen-katalytische Umsetzung von Synthesegas in geeigneten Synthesereaktoren. Synthesegase können unterschiedliche Gasgemische sein, die unter anderem Wasserstoff und Kohlenoxide enthalten. Der Reaktor ist meist als ein stehender Festrohrwärmetauscher ausgeführt.

Bekannt sind auch zweistufige Verfahren zur Herstellung von Methanol. Dabei wird Synthesegas einem wassergekühlten Reaktor und anschließend einem gasgekühlten Reaktor zugeführt. Mit einem kupferbasierten Festbett-Katalysator wird das Synthesegas zu Methanol umgesetzt.

Bei einem wassergekühlten Reaktor befindet sich der Katalysator innerhalb der Rohre, umgeben von Wasser oder Wasserdampf auf der Mantelseite. Die Rohre werden über Rohrplatten und Stützbleche im Reaktor mechanisch fixiert.

Die Kühlung im wassergekühlten Reaktor erfolgt über eine Wärmeabgabe ins Wasser, wobei Dampf entstehen kann. Das Dampf-Wasser-Gemisch steigt an den Rohren auf. Die Stützbleche müssen die Fixierung der Rohre sicherstellen. Bekannt sind Stützbleche, die versetzt im Reaktorbehälter eingesetzt sind, so dass das Kühlmittel mäanderförmig im Reaktorbehälter um die Stützbleche strömt.

Abhängig von den gewählten Geometrien der wärmeübertragenden Komponenten und der mechanischen Elemente der Kühlmittelseite wird ein Druckverlust auf der Kühlmittelseite im Wasserdampf erzeugt. Vor allem lange Reaktorrohre führen zu einem höheren Druckverlust.

Häufig sind die äußeren Abmessungen des Reaktors beim Transport beschränkt, so dass dies zu langen, schlanken Reaktoren führt. Daraus folgt jedoch ein erhöhter Druckverlust auf der Kühlmittelseite. Durch die lange Reaktorform werden weitere Stützplatten notwendig, um ein Durchhängen der Rohre bei Transport, sowie Durchbiegen, Knicken und Schwingen im Betrieb zu vermeiden.

Aufgabe der vorliegenden Erfindung ist es, ausgehend vom beschriebenen Stand der Technik die Hinderniswirkung der Stützplatte zu reduzieren und gleichzeitig ein Knicken, Durchbiegen oder Schwingen der Rohre zu verhindern.

Diese Aufgabe wird gelöst mit den Gegenständen der unabhängigen Ansprüche. Weitere vorteilhafte Ausgestaltungen sind in den abhängigen Ansprüchen angegeben. Die in den Ansprüchen und in der Beschreibung dargestellten Merkmale sind in beliebiger, technologisch sinnvoller Weise miteinander kombinierbar.

Erfindungsgemäß wird eine Vorrichtung vorgestellt, die einen Reaktorbehälter, ein Rohrbündel mit mehreren Rohren, eine erste Stützplatte und eine zweite Stützplatte umfasst. Das Rohrbündel ist im Reaktorbehälter angeordnet. Das Rohrbündel umfasst eine erste Rohrgruppe, eine zweite Rohrgruppe und eine dritte Rohrgruppe.

Die erste Stützplatte und die zweite Stützplatte sind quer zu einer Längsachse des Reaktorbehälters im Reaktorbehälter angeordnet.

Jedes der Rohre der ersten Rohrgruppe ist durch eine jeweilige Rohröffnung der ersten Stützplatte geführt, wobei die erste Stützplatte mehrere Aussparungen zum Fluidaustausch aufweist. Jedes der Rohre der zweiten Rohrgruppe ist durch eine jeweilige der Aussparungen in der ersten Stützplatte geführt.

Jedes der Rohre der zweiten Rohrgruppe ist durch eine jeweilige Rohröffnung der zweiten Stützplatte geführt, wobei die zweite Stützplatte mehrere Aussparungen zum Fluidaustausch aufweist. Jedes der Rohre der ersten Rohrgruppe ist durch eine jeweilige der Aussparungen in der zweiten Stützplatte geführt.

Jedes der Rohre der dritten Rohrgruppe ist durch eine jeweilige Rohröffnung der ersten Stützplatte geführt und durch die zweite Stützplatte hindurchgeführt. Die erste Stützplatte stützt die Rohre der ersten Rohrgruppe und der dritten Rohrgruppe in den Rohröffnungen der ersten Stützplatte quer zur Längsrichtung der Rohre. Die zweite Stützplatte stützt die Rohre der zweiten Rohrgruppe in den Rohröffnungen der zweiten Stützplatte.

Die Vorrichtung umfasst einen Reaktorbehälter und ein darin angeordnetes Rohrbündel mit mehreren Rohren. Durch die Rohre kann ein erstes Medium strömen. Außerhalb der Rohre kann sich ein zweites Medium in dem Reaktorbehälter befinden. Das zweite Medium kann durch Zwischenräume zwischen den Rohren strömen. Zwischen dem ersten Medium und dem zweiten Medium kann ein Wärmetausch stattfinden. Das erste Medium und das zweite Medium können in einander entgegengesetzte Richtungen strömen. In dem Fall wird die Vorrichtung im Gegenstrombetrieb betrieben. Das erste Medium und das zweite Medium können aber auch in die gleiche Richtung strömen. Im Falle der Methanolsynthese kann das erste Medium beispielsweise die Reaktionsedukte enthalten und das zweite Medium ein Kühlmedium sein, oder umgekehrt.

Vorzugsweise ist der Reaktorbehälter ein länglicher Hohlkörper. Der Reaktorbehälter kann ein zylindrischer Metallbehälter sein, der dazu geeignet ist, das Rohrbündel zu umfassen. Der Reaktorbehälter kann Anschlüsse und Verbindungen aufweisen, so dass das Rohrbündel fluidtechnisch angeschlossen werden kann. Weiterhin kann der Reaktorbehälter Anschlüsse und Verbindungen aufweisen, so dass in einen Mantelraum außerhalb und zwischen den Rohren ein Kühlmedium oder sonstiges Medium in den Reaktorbehälter eingeleitet und wieder abgeleitet werden kann. Der Mantelraum kann fluidtechnisch angeschlossen werden.

Das Rohrbündel umfasst mehrere Rohre. Das Rohrbündel umfasst vorzugsweise mindestens 6 Rohre. Bevorzugt umfasst das Rohrbündel mindestens 16 Rohre. Besonders bevorzugt umfasst das Rohrbündel sogar mindestens 1000 Rohre.

Das Rohrbündel umfasst eine erste Rohrgruppe, eine zweite Rohrgruppe und eine dritte Rohrgruppe. Jede der Rohrgruppen umfasst jeweils mehrere der Rohre. Es ist möglich, aber nicht erforderlich, dass das Rohrbündel auch ein oder mehrere Rohre umfasst, welche zu keiner der Rohrgruppen gehören. Jedes der Rohre gehört entweder zur ersten Rohrgruppe, zur zweiten Rohrgruppe, zur dritten Rohrgruppe oder zu keiner der Rohrgruppen. Auch wenn es Rohre gibt, die zu keiner der Rohrgruppen gehören, gibt es jedenfalls kein Rohr, welches zu mehreren der Rohrgruppen zugleich gehört. Die Rohrgruppen sind allein durch die hierin beschriebenen Eigenschaften definiert. Es ist nicht erforderlich, dass die Zuordnung der Rohre zu den Rohrgruppen darüber hinaus an strukturellen Merkmalen der Vorrichtung zu erkennen ist.

Bevorzugt umfasst die erste Rohrgruppe mindestens sechs Rohre und/oder die zweite Rohrgruppe umfasst mindestens sechs Rohre und/oder die dritte Rohrgruppe umfasst mindestens zwei Rohre. Besonders bevorzugt ist die Anzahl an Rohren der ersten Rohrgruppe zur Anzahl an Rohren der dritten Rohrgruppe im Verhältnis 3:1 und/oder die Anzahl an Rohren der zweiten Rohrgruppe zur Anzahl an Rohren der dritten Rohrgruppe ist im Verhältnis 3:1. Die "und"-Ausführungen sind bevorzugt.

Die Rohre können längliche zylindrische Hohlkörper mit einer gleichmäßigen Wanddicke sein. Die Wanddicke der Rohre beträgt vorzugsweise höchstens 5 mm, bevorzugt höchstens 2,5 mm und besonders bevorzugt höchstens 0,1 mm. Insbesondere kann die Wanddicke der Rohre einer für die Anwendung genormten Wanddicke entsprechen. Die Rohre können insbesondere aus einem metallischen Material gefertigt sein. Bevorzugt ist das Material wärmeleitend.

Vorzugsweise beinhalten einige der Rohre oder alle Rohre einen Katalysator. Im Falle der Methanolsynthese können so beispielsweise die Reaktionsedukte durch die Rohre geleitet werden und in den Rohren mit dem Katalysator zur Reaktion gebracht werden, so dass Methanol entsteht.

Die Reaktionsedukte können insbesondere in Form eines Synthesegases bereitgestellt werden. Vorzugsweise sind Wasserstoff, Kohlenmonoxid und Kohlenstoffdioxid Reaktionsedukte. Bevorzugt ist ein Gemisch aus Wasserstoff und Kohlenmonoxid. Besonders bevorzugt ist ein Gemisch aus Wasserstoff und Kohlenstoffdioxid. Weiterhin kann das Synthesegas Inertgase enthalten. Insbesondere kann das Synthesegas Methan als Inertgas enthalten. Bevorzugt enthält das Synthesegas Stickstoff als Inertgas. Das Synthesegas kann auf einer Einlassseite in die Rohre geleitet werden und mit dem Katalysator im Rohr reagieren. Im Falle einer exothermen Reaktion kann bei der Reaktion entstehende Wärme über den Rohrmantel abgeleitet werden. Ein Kühlmittel kann die Wärme, bevorzugt konvektiv, von der Mantelseite abführen. Das im Rohr entstandene Produkt der Reaktion kann zusammen mit dem verbleibenden Synthesegas auf der Auslassseite abgeführt werden.

Alternativ kann ein Synthesegas mit den Reaktionsedukten auch außerhalb der Rohre durch den Reaktorbehälter geleitet werden. Insbesondere in dem Fall kann eine Katalysatorschüttung auf der Mantelseite im Mantelraum vorgesehen sein. Das Synthesegas kann dann durch die Katalysatorschüttung geführt werden. Ein Kühlmittel kann auf einer Einlassseite in die Rohre geleitet werden. Die durch die Reaktion erzeugte Wärme kann über die Mantelseite der Rohre an das Kühlmittel in den Rohren übertragen werden. Das erwärmte Kühlmittel kann auf einer Auslassseite der Rohre abgeführt werden.

Weiterhin umfasst die Vorrichtung eine erste Stützplatte und eine zweite Stützplatte. Die erste Stützplatte und/oder die zweite Stützplatte können als ein Blech ausgebildet sein. Vorzugsweise haben die erste Stützplatte und/oder die zweite Stützplatte eine Dicke von mindestens 7 mm. Bevorzugt ist die Dicke der ersten Stützplatte und/oder der zweiten Stützplatte höchstens halb so groß wie ein Durchmesser der Rohre. Die optimale Dicke der Stützplatte kann darüber hinaus über Berechnungen ermittelt werden. Haben nicht alle Rohre den gleichen Durchmesser, ist die Dicke der ersten Stützplatte und/oder der zweiten Stützplatte vorzugsweise höchstens halb so groß wie der größte Durchmesser der Rohre des Rohrbündels.

Die erste Stützplatte und die zweite Stützplatte sind quer zu einer Längsachse des Reaktorbehälters im Reaktorbehälter angeordnet. Insbesondere können die erste Stützplatte und die zweite Stützplatte im Reaktorbehälter befestigt sein.

Die Längsachse des Reaktorbehälters ist bevorzugt entlang der länglichen Richtung des Hohlkörpers ausgerichtet. Insbesondere ist die erste Stützplatte und/oder die zweite Stützplatte senkrecht zur Längsachse des Reaktorbehälters im Reaktorbehälter angeordnet. Vorzugsweise ist der Reaktorbehälter und/oder das Rohrbündel vertikal ausgerichtet. Die "und"-Ausführungen sind bevorzugt.

Vorzugsweise weist die erste Stützplatte einen Abstand entlang der Längsachse des Reaktorbehälters von höchstens 500 mm zur zweiten Stützplatte auf.

Jedes der Rohre der ersten Rohrgruppe ist durch eine jeweilige Rohröffnung der ersten Stützplatte geführt, wobei die erste Stützplatte mehrere Aussparungen zum Fluidaustausch aufweist. Jedes der Rohre der zweiten Rohrgruppe ist durch eine jeweilige der Aussparungen in der ersten Stützplatte geführt.

Jedes der Rohre der zweiten Rohrgruppe ist durch eine jeweilige Rohröffnung der zweiten Stützplatte geführt, wobei die zweite Stützplatte mehrere Aussparungen zum Fluidaustausch aufweist. Jedes der Rohre der ersten Rohrgruppe ist durch eine jeweilige der Aussparungen in der zweiten Stützplatte geführt.

Jedes der Rohre der dritten Rohrgruppe ist durch eine jeweilige Rohröffnung der ersten Stützplatte geführt und durch die zweite Stützplatte hindurchgeführt.

Die Rohröffnungen der ersten Stützplatte sind den Rohren der ersten und dritten Rohrgruppe zugeordnet. Die Rohröffnungen der zweiten Stützplatte sind den Rohren der zweiten Rohrgruppe zugeordnet.

Die erste Stützplatte und die zweite Stützplatte können den Fluidaustausch im Mantelraum des Reaktorbehälters beeinträchtigen. Um diesen Effekt so gering wie möglich zu halten, weisen die erste Stützplatte und die zweite Stützplatte jeweils mehrere Aussparungen auf. Der Fluidaustausch findet insbesondere zwischen Bereichen des Reaktorbehälters statt, zwischen denen die erste Stützplatte und die zweite Stützplatte angeordnet sind.

Ein freier Strömungsquerschnitt im Reaktorbehälter kann durch die Aussparungen in der ersten Stützplatte und in der zweiten Stützplatte bestimmt werden. Der freie Strömungsquerschnitt ist der Querschnitt eines Rohres oder Kanals, durch den ein Medium strömt. Der freie Strömungsquerschnitt bezeichnet also eine Fläche, durch die ein Medium strömen kann. Je größer die kleinste Gesamtfläche aller Aussparungen der ersten Stützplatte oder der zweiten Stützplatte, desto größer ist der freie Strömungsquerschnitt. Anderseits kann der freie Strömungsquerschnitt auch über die Teilung bestimmt werden.

Die Teilung beschreibt den Abstand von Rohr zu Rohr. Je mehr Abstand zwischen Rohren ist, desto größer ist der freie Strömungsquerschnitt ohne eine Stützplatte. Die Teilung kann somit eine maximale Fläche der Aussparungen definieren.

Die erste Stützplatte stützt die Rohre der ersten Rohrgruppe und der dritten Rohrgruppe in den Rohröffnungen der ersten Stützplatte quer zur Längsrichtung der Rohre. Die zweite Stützplatte stützt die Rohre der zweiten Rohrgruppe in den Rohröffnungen der zweiten Stützplatte.

Die Rohre der ersten Rohrgruppe sind durch eine jeweilige Rohröffnung der ersten Stützplatte geführt. Jedes Rohr hat dabei eine eigene Rohröffnung. Dadurch werden die Rohre der ersten Rohrgruppe mit der ersten Stützplatte gestützt. Die Rohre der ersten Rohrgruppe sind zudem durch eine jeweilige Aussparung der zweiten Stützplatte geführt. Die erste Rohrgruppe umfasst also all die Rohre, die durch eine der Aussparungen der zweiten Stützplatte geführt werden. Die Rohre der ersten Rohrgruppe werden im Allgemeinen nicht durch die zweite Stützplatte gestützt. Stattdessen passieren diese Rohre die zweite Stützplatte lediglich, indem die Rohre durch die entsprechenden Aussparungen geführt sind. Die Aussparungen in der zweiten Stützplatte dienen dem Fluidaustausch.

Die Rohre der zweiten Rohrgruppe sind durch eine jeweilige Rohröffnung der zweiten Stützplatte geführt. Jedes Rohr hat dabei eine eigene Rohröffnung. Dadurch werden die Rohre der zweiten Rohrgruppe mit der zweiten Stützplatte gestützt. Die Rohre der zweiten Rohrgruppe sind zudem durch eine jeweilige Aussparung der ersten Stützplatte geführt. Die zweite Rohrgruppe umfasst also jeweils all die Rohre, die gemeinsam durch eine der Aussparungen der ersten Stützplatte geführt werden. Die Rohre der zweiten Rohrgruppe werden im Allgemeinen nicht durch die erste Stützplatte gestützt. Stattdessen passieren diese Rohre die erste Stützplatte lediglich, indem die Rohre durch die entsprechenden Aussparungen geführt sind. Die Aussparungen in der ersten Stützplatte dienen dem Fluidaustausch.

Die Rohre der dritten Rohrgruppe sind durch eine jeweilige Rohröffnung der ersten Stützplatte geführt und durch die zweite Stützplatte hindurchgeführt. Jedes Rohr der dritten Rohrgruppe hat dabei eine eigene Rohröffnung in der ersten Stützplatte. Dadurch werden die Rohre der dritten Rohrgruppe mit der ersten Stützplatte gestützt.

Dies hat den Vorteil, dass die Rohre des Rohrbündels sich individuell thermisch ausdehnen können, aber Schwingungen durch bestimmte Strömungszustände und ein Knicken der Rohre vermieden werden können. Ein weiterer Vorteil ist, dass alle Rohre des Rohrbündels von einer ersten Stützplatte zusammen mit einer zweiten Stützplatte gestützt sind.

Dass die Rohre der dritten Rohrgruppe durch die zweite Stützplatte hindurchgeführt sind, kann auf verschiedene Weisen realisiert sein. Beispielsweise können die Rohre der dritten Rohrgruppe durch eine jeweilige Rohröffnungen in der zweiten Stützplatte geführt sein, wobei die zweite Stützplatte die Rohre der dritten Rohrgruppe in den Rohröffnungen der zweiten Stützplatte stützt. Alternativ können die Rohre der dritten Rohrgruppe durch eine jeweilige Aussparung zum Fluidaustausch in der zweiten Stützplatte geführt sein. Auch können mehrere oder alle Rohre der dritten Rohrgruppe gemeinsam durch eine Aussparung zum Fluidaustausch in der zweiten Stützplatte geführt sein.

Vorzugsweise sind die Rohre des Rohrbündels in axialer Richtung in der jeweiligen Rohröffnung der ersten Stützplatte und der zweiten Stützplatte nicht fixiert. Die Rohre der ersten Rohrgruppe und der dritten Rohrgruppe sind in dem Fall in axialer Richtung durch eine jeweilige Rohröffnung der ersten Stützplatte axial bewegbar. Die Rohre der zweiten Rohrgruppe sind in demselben Fall in axialer Richtung durch eine jeweilige Rohröffnung der zweiten Stützplatte axial bewegbar. Lokal unterschiedliche Temperaturen können lokal unterschiedliche Ausdehnungen der Rohre zur Folge haben. Dadurch, dass die Rohre axial bewegbar sind, werden einzelne Rohre nicht durch eine der Stützplatten in ihrer individuellen Ausdehnung gestört. Bewegungen quer zur Längsachse werden jedoch minimiert.

Die Form der ersten Stützplatte und der zweiten Stützplatte kann der des Reaktorbehälters angepasst sein. Vorzugsweise weist die erste Stützplatte und/oder die zweite Stützplatte eine rechteckige Form mit abgerundeten Ecken auf. Besonders bevorzugt weist die erste Stützplatte und/oder die zweite Stützplatte eine elliptische Form, insbesondere eine kreisförmige Form auf. Die "und"-Ausführungen sind bevorzugt.

Die Vorrichtung hat den Vorteil, dass die Hinderniswirkung der ersten Stützplatte und der zweiten Stützplatte minimiert wird und gleichzeitig ein Knicken, Durchbiegen oder Schwingen der Rohre während des Transports oder während des Betriebs verhindert wird.

In einer bevorzugten Ausführungsform der Vorrichtung sind die Rohröffnungen der ersten Stützplatte jeweils von einem ringförmigen Steg umgeben, wobei die Stege jeweils eine minimale Breite haben, welche bei zumindest einigen der Stege gleich groß ist. Zusätzlich oder alternativ sind die Rohröffnungen der zweiten Stützplatte jeweils von einem ringförmigen Steg umgeben, wobei die Stege jeweils eine minimale Breite haben, welche bei zumindest einigen der Stege gleich groß ist.

Die erste Stützplatte und die zweite Stützplatte sollen einerseits so stabil ausgebildet sein, dass die Rohre ausreichend gestützt werden. Dies kann dadurch erreicht werden, dass die Stege eine ausreichende Breite haben. Andererseits soll die Stützplatte den Fluidaustausch möglichst wenig beeinträchtigen. Dies kann dadurch erreicht werden, dass die Stege möglichst klein gehalten sind. Durch Abwägung der beiden genannten Bedingungen kann bestimmt werden, wie breit ein Steg an seiner engsten Stelle optimalerweise sein sollte, wie groß also die minimale Breite eines Steges optimalerweise sein sollte. In der vorliegenden Ausführungsform haben zumindest einige der Stege, vorzugsweise sogar alle der Stege die gleiche minimale Breite. Damit kann für alle diese Stege ein bestmöglicher Kompromiss zwischen Stabilität und Fluidaustausch erzielt werden.

Vorzugsweise sind die ringförmigen Stege der ersten Stützplatte über einen jeweiligen ersten Übergang verbunden und/oder die ringförmigen Stege der zweiten Stützplatte sind über einen jeweiligen zweiten Übergang verbunden. Die "und"-Ausführung ist bevorzugt.

In einer weiteren bevorzugten Ausführungsform der Vorrichtung ist jedes der Rohre der dritten Rohrgruppe durch eine jeweilige Rohröffnung der zweiten Stützplatte geführt, welche kongruent zu der Rohröffnung der ersten Stützplatte liegt, durch welche das jeweilige Rohr der dritten Rohrgruppe geführt ist.

Die zweite Stützplatte stützt insbesondere die Rohre der zweiten Rohrgruppe und der dritten Rohrgruppe in den Rohröffnungen der zweiten Stützplatte quer zur Längsrichtung der Rohre. Dadurch sind die Rohröffnungen der zweiten Stützplatte den Rohren der zweiten und dritten Rohrgruppe zugeordnet.

Die Rohre der dritten Rohrgruppe sind durch eine jeweilige Rohröffnung der ersten Stützplatte geführt. Jedes Rohr hat dabei eine eigene Rohröffnung. Dadurch werden die Rohre der dritten Rohrgruppe mit der ersten Stützplatte gestützt. Weiterhin sind die Rohre der dritten Rohrgruppe durch eine jeweilige Rohröffnung der zweiten Stützplatte geführt. Jedes Rohr hat dabei eine eigene Rohröffnung. Dadurch werden die Rohre der dritten Rohrgruppe weiterhin mit der zweiten Stützplatte gestützt. Die dritten Rohrgruppe umfasst also jeweils all die Rohre, die sowohl durch eine Rohröffnung in der ersten Stützplatte als auch durch eine Rohröffnung in der zweiten Stützplatte geführt werden.

Dies hat eine Kraftkopplung von erster Rohrgruppe zur zweiten Rohrgruppe über die dritte Rohrgruppe mit Hilfe der ersten Stützplatte und der zweiten Stützplatte zur Folge.

Der Vorteil dieser Ausführungsform ist, dass die Rohre der ersten Rohrgruppe und die Rohre der zweiten Rohrgruppe über die Rohre der dritten Rohrgruppe mechanisch gekoppelt werden. Dies hat eine erhöhte Stabilität des Rohrbündels im Reaktorbehälter zur Folge und bessere

Dämpfungseigenschaften gegenüber Schwingungen der Rohre im Betrieb. Weiterhin kann ein Knicken der Rohre beim Transport des Reaktorbehälters vorteilhafter verhindert werden.

In einer weiteren bevorzugten Ausführungsform der Vorrichtung weisen die Aussparungen der ersten Stützplatte jeweils eine Sternform mit sechs Zacken auf. Zusätzlich oder alternativ weisen die Aussparungen der zweiten Stützplatte jeweils eine Sternform mit sechs Zacken auf.

Diese Ausführungsform hat den Vorteil, dass der freie Strömungsquerschnitt dadurch möglichst groß ist. Dies hat einen positiven Einfluss auf die Hinderniswirkung der ersten Stützplatte und der zweiten Stützplatte. Dadurch kann der Druckverlust reduziert werden.

In einer weiteren bevorzugten Ausführungsform der Vorrichtung ist jedes Rohr der dritten Rohrgruppe von jeweils drei Rohren der ersten Rohrgruppe umgeben. Zusätzlich oder alternativ ist jedes Rohr der dritten Rohrgruppe von jeweils drei Rohren der zweiten Rohrgruppe umgeben.

Dabei sind insbesondere jeweils die nächstliegenden Rohre der jeweiligen Rohrgruppen gemeint. Diese beschriebene Gruppierung von Rohren der ersten Rohrgruppe, der zweiten Rohrgruppe und der dritten Rohrgruppe kann auch als ein Muster bezeichnet werden. Dieses Muster kann sich im Rohrbündel wiederholen.

Mit Hilfe dieses Musters können Kräfte quer zur Längsachse, welche auf eine der Rohrgruppen wirken, gleichmäßig gemäß der beschriebenen Kraftkopplung von erster Rohrgruppe über die dritte Rohrgruppe zur zweiten Rohrgruppe abgeleitet sowie verteilt werden und umgekehrt.

Hierdurch wird eine gleichmäßigere Kraftverteilung von Kräften quer zur Längsachse durch beispielsweise Strömungen oder durch die Schwerkraft beim Transport des Reaktorbehälters ermöglicht.

In einer weiteren bevorzugten Ausführungsform der Vorrichtung bilden die erste Stützplatte und die zweite Stützplatte zusammen ein Stützelement aus.

Jedes der Rohre ist also durch die erste Stützplatte und durch die zweite Stützplatte gestützt. Die erste Stützplatte und die zweite Stützplatte werden insoweit als ein Stützelement aufgefasst. Das Stützelement bewirkt also die gewünschte Stützung aller Rohre. Vorzugsweise ist die erste Stützplatte unmittelbar an der zweiten Stützplatte angeordnet. Die beschriebenen Vorteile durch die erste Stützplatte zusammen mit der zweiten Stützplatte werden bereits erzielt, wenn die Vorrichtung ein einzelnes Stützelement aufweist.

In einer bevorzugten Weiterbildung der vorherigen Ausführungsform sind in dem Reaktorbehälter mehrere der Stützelemente angeordnet, welche entlang der Längsachse des Reaktorbehälters zueinander versetzt sind.

Es ist aber auch möglich und sogar bevorzugt, dass die Vorrichtung mehrere Stützelemente aufweist, welche jeweils eine erste Stützplatte und eine zweite Stützplatte aufweisen. Die Stützelemente sind vorzugsweise jeweils wie hierin beschrieben ausgebildet.

Der Vorteil mehrerer Stützelemente ist, dass die Rohre des Rohrbündels besonders sicher gehalten werden. Ein Knicken, Durchbiegen oder Schwingen der Rohre wird vorteilhaft noch weiter reduziert.

Als ein weiterer Aspekt der Erfindung wird eine Anordnung zur Methanolsynthese vorgestellt. Die Anordnung umfasst die beschriebene Vorrichtung. Die beschriebenen Vorteile und Merkmale der Vorrichtung sind auf die Anordnung anwendbar und übertragbar.

Zum Erzeugen von Methanol kann ein Synthesegas durch Umsetzen an Katalysatoren zu Methanol umgesetzt werden. Das Synthesegas kann Reaktionsedukte für die Methanolsynthese enthalten.

Neben der beschriebenen Vorrichtung umfasst die Anordnung vorzugsweise eine Quelle für Reaktionsedukte für die Methanolsynthese. Die Anordnung kann auch mehrere wie beschrieben ausgebildete Vorrichtungen aufweisen, welche beispielsweise in Reihe geschaltet sind.

Als ein weiterer Aspekt der Erfindung wird ein Verfahren vorgestellt. In dem Verfahren werden Reaktionsedukte für die Methanolsynthese durch die Rohre des Rohrbündels geleitet. Ein Kühlmedium wird außerhalb der Rohre des Rohrbündels durch den Reaktorbehälter geleitet. Vorzugsweise ist das Kühlmedium flüssiges Wasser und/oder Wasserdampf.

In einer alternativen Ausführung des Verfahrens können die Reaktionsedukte für die Methanolsynthese um die Rohre des Rohrbündels durch den Reaktorbehälter geleitet werden. Ein Kühlmedium wird dann durch die Rohre des Rohrbündels geleitet. Vorzugsweise ist das Kühlmedium gasförmig.

Die beschriebenen Vorteile und Merkmale der Vorrichtung sind auf das Verfahren anwendbar und übertragbar, und umgekehrt. Die Vorrichtung ist vorzugsweise zum Betrieb gemäß dem beschriebenen Verfahren eingerichtet. Das Verfahren wird vorzugsweise mit der Vorrichtung durchgeführt.

Als ein weiterer Aspekt der Erfindung wird eine Verwendung vorgestellt. Die beschriebene Vorrichtung wird dabei zur Methanolsynthese verwendet. Reaktionsedukte werden in der Vorrichtung zu Methanol umgesetzt.

Die beschriebenen Vorteile und Merkmale der Vorrichtung, der Anordnung und des Verfahrens sind auf die Verwendung anwendbar und übertragbar, und umgekehrt.

Vorzugsweise wird die Vorrichtung für die wassergekühlte Methanolsynthese verwendet. Alternativ ist bevorzugt, dass die Vorrichtung für die gasgekühlte Methanolsynthese verwendet wird.

In der wassergekühlten Methanolsynthese werden die Rohre mit Wasser gekühlt, während ein Synthesegas in den Rohren zu Methanol umgesetzt wird. Das Wasser führt die Wärme von den Rohren ab und verdampft dabei teilweise. Die aufsteigenden Dampfblasen können durch die Aussparungen der ersten Stützplatte und durch die Aussparungen der zweiten Stützplatte, oder umgekehrt, strömen.

In der gasgekühlten Methanolsynthese werden die Rohre mit einem kühleren Gas in den Rohren gekühlt. Das Synthesegas wird außerhalb der Rohre zu Methanol umgesetzt. Dabei wird die Wärme an die Rohre übertragen und durch das Gas in den Rohren abgeführt. Das Synthesegas kann durch die Aussparungen der ersten Stützplatte und durch die Aussparungen der zweiten Stützplatte, oder umgekehrt, strömen.

Die Erfindung wird nachfolgend anhand der Figuren näher erläutert. Die Figuren zeigen ein besonders bevorzugtes Ausführungsbeispiel, auf das die Erfindung jedoch nicht begrenzt ist. Die Figuren und die darin dargestellten Größenverhältnisse sind nur schematisch. Es zeigen:
- Fig. 1:: eine schematische Ansicht einer erfindungsgemäßen Anordnung zur Methanolsynthese,
- Fig. 2:: eine perspektivische Detailansicht einer ersten Ausgestaltung einer ersten Stützplatte und einer zweiten Stützplatte, wie sie bei der Anordnung aus Fig. 1 verwendet werden können.

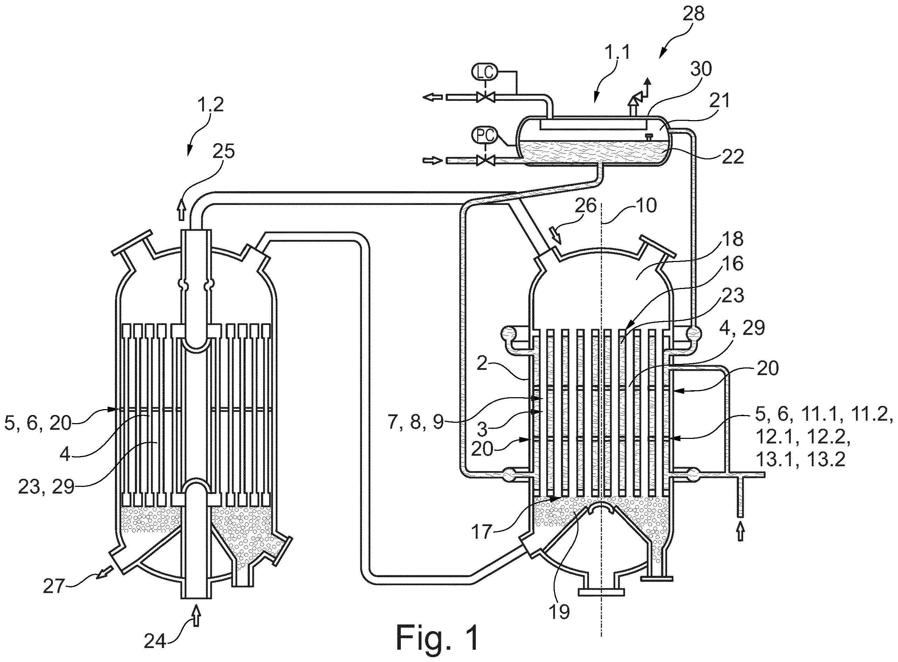

Fig. 1 zeigt eine schematische Ansicht einer erfindungsgemäßen Anordnung 28 zur Methanolsynthese. Dabei wird Synthesegas durch zwei in Serie geschaltete Vorrichtungen 1.1; 1.2 geleitet. Die Vorrichtungen 1.1;1.2 werden zur Methanolsynthese verwendet. Vorgeheiztes Synthesegas wird durch einen Gaseinlass 26 der ersten Vorrichtung 1.1 zugeführt.

Die erste Vorrichtung 1.1 umfasst einen Reaktorbehälter 2, ein Rohrbündel 3 mit mehreren Rohren 4, eine erste Stützplatte 5 und eine zweite Stützplatte 6. Das Rohrbündel 3 ist im Reaktorbehälter 2 angeordnet, wobei das Rohrbündel 3 eine erste Rohrgruppe 7, eine zweite Rohrgruppe 8 und eine dritte Rohrgruppe 9 umfasst. Die erste Stützplatte 5 und die zweite Stützplatte 6 sind quer zu einer Längsachse 10 des Reaktorbehälters 2 im Reaktorbehälter 2 angeordnet. Jedes der Rohre 4 der ersten Rohrgruppe 7 ist durch eine jeweilige Rohröffnung 11.1 der ersten Stützplatte 5 geführt und die erste Stützplatte 5 weist mehrere Aussparungen 13.1 zum Fluidaustausch auf. Jedes der Rohre der zweiten Rohrgruppe 8 ist durch eine jeweilige der Aussparungen 13.1 in der ersten Stützplatte 5 geführt, wobei jedes der Rohre 4 der zweiten Rohrgruppe 8 durch eine jeweilige Rohröffnung 12.1 der zweiten Stützplatte 6 geführt ist, und wobei die zweite Stützplatte 6 mehrere Aussparungen 13.2 zum Fluidaustausch aufweist, wobei jedes der Rohre der ersten Rohrgruppe 7 durch eine jeweilige der Aussparungen 13.2 in der zweiten Stützplatte 6 geführt ist, wobei jedes der Rohre 4 der dritten Rohrgruppe 9 durch eine jeweilige Rohröffnung 11.2 der ersten Stützplatte 5 geführt ist, wobei die erste Stützplatte 5 die Rohre 4 der ersten Rohrgruppe 7 und der dritten Rohrgruppe 9 in den Rohröffnungen 11.1, 11.2 der ersten Stützplatte 5 quer zur Längsrichtung der Rohre 4 stützt und die zweite Stützplatte 6 die Rohre 4 der zweiten Rohrgruppe 8 in den Rohröffnungen 12.1 der zweiten Stützplatte 6 stützt.

Jedes der Rohre 4 der dritten Rohrgruppe 9 ist durch eine jeweilige Rohröffnung 12.2 der zweiten Stützplatte 6 geführt, welche kongruent zu der Rohröffnung 11.2 der ersten Stützplatte 5 liegt, durch welche das jeweilige Rohr 4 der dritten Rohrgruppe 9 geführt ist.

Die erste Stützplatte 5 und die zweite Stützplatte 6 bilden zusammen ein Stützelement 20 aus. In dem Reaktorbehälter sind mehrere der Stützelemente 20 angeordnet, welche entlang der Längsachse 10 des Reaktorbehälters 2 zueinander versetzt sind.

Die Rohre 4 sind an einem ersten Ende 16 und an einem zweiten Ende 17 mit einer jeweiligen Rohrendplatte verbunden. Der Reaktorbehälter 2 und die Rohre 4 des Rohrbündels 3 sind aufrecht ausgerichtet. Das erste Ende 16 ist oben und das zweite Ende 17 ist unten im Reaktorbehälter 2 angeordnet. Das Synthesegas wird in einem Verteiler 18 auf die Rohre 4 des Rohrbündels 3 verteilt.

In den Rohren 4 befindet sich ein Katalysator 29. Das Synthesegas enthält Reaktionsedukte. Die Reaktionsedukte für die Methanolsynthese werden durch die Rohre 4 des Rohrbündels 3 geleitet. Dabei wird das Synthesegas in den Rohren 4 teilweise zu Methanol umgesetzt. Ein Kühlmedium wird außerhalb der Rohre 4 des Rohrbündels 3 durch den Reaktorbehälter 2 geleitet. Die aus der exothermen Reaktion im Rohr 4 freiwerdende Wärme wird über die Rohrwand auf das in einem Mantelraum 23 befindliche Kühlmedium übertragen. Während der Katalysator 29 im Rohr 4 durch diesen Prozess gekühlt wird, wird dem Kühlmedium Energie zugeführt. Als Kühlmedium dient Wasser, welches dem Mantelraum 23 am zweiten Ende 17 zugeführt wird. Entstehende Dampfblasen und das siedende zweiphasige Wassergemisch strömen aufgrund von Dichteunterschieden vertikal nach oben. Dabei strömt der Wasserdampf durch die Aussparungen 13.1 der ersten Stützplatte 5 und durch die Aussparungen 13.2 der zweiten Stützplatte 6 mit geringen Druckverlusten. Der Wasserdampf wird oben gesammelt und einem Wasser-Kondensator 30 zugeführt. In dem Reservoir des Kondensators 30 befinden sich gesättigter Dampf 21 und Wasser 22 am bzw. leicht unter dem Siedepunkt. Das Wasser 22 wird der ersten Vorrichtung 1.1 am zweiten Ende 17 zugeführt und auf den Mantelraum 23 des Reaktorbehälters 2 verteilt. Die Kühlung funktioniert damit nach dem Thermosiphoneffekt.

Produktgas und das teilweise nicht reagierte Synthesegas werden aus dem Rohrbündel 3 in einem Sammler 19 gesammelt. Das Gasgemisch strömt anschließend zu der zweiten Vorrichtung 1.2.

In der zweiten Vorrichtung 1.2, welche der ersten Vorrichtung 1.1 nachgeschaltet ist, wird Synthesegas aus einem Synthesegaseinlass 24 vorgeheizt.

Die zweite Vorrichtung 1.2 ist teilweise analog zur ersten Vorrichtung 1.1 aufgebaut.

Im Unterschied zur ersten Vorrichtung 1.1, befindet sich in der zweiten Vorrichtung 1.2 zwischen den Rohren 4 der zweiten Vorrichtung 1.2 im Mantelraum 23 ein als Katalysatorschüttung vorliegender Katalysator 29.

Das Synthesegas, welches die Reaktionsedukte umfasst, wird für die Methanolsynthese außerhalb der Rohre 4 des Rohrbündels 3 durch den Reaktorbehälter 2 durch den Mantelraum 23 geleitet und ein Kühlmedium wird durch die Rohre 4 des Rohrbündels 3 geleitet. Dabei strömt das Synthesegas im Mantelraum 23 durch die Aussparungen 13.1 der ersten Stützplatte 5 und durch die Aussparungen 13.2 der zweiten Stützplatte 6 mit geringen Druckverlusten nach unten zu einem Produktgasauslass 27.

Die aus der exothermen Reaktion im Mantelraum 23 freiwerdende Wärme wird über die Rohrwand auf das im Rohr 4 befindliche Kühlmedium übertragen.

Während der Katalysator 29 im Mantelraum 23 durch diesen Prozess gekühlt wird, wird dem Kühlmedium Energie zugeführt. Als Kühlmedium dient Synthesegas, welches den Rohren 4 über den Synthesegaseinlass 24 zugeführt wird. Durch die zugeführte Energie erwärmt sich das Synthesegas aus dem Synthesegaseinlass 24 und wird als vorgeheiztes Synthesegas 25 zum Gaseinlass 26 der ersten Vorrichtung 1.1 geführt.

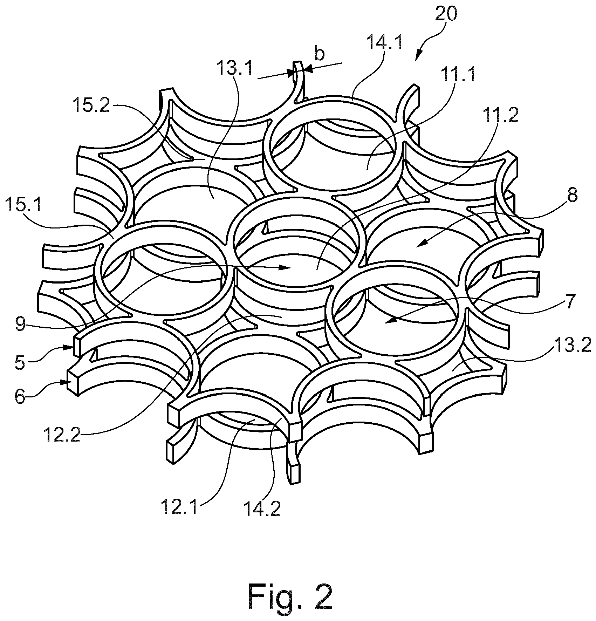

Fig. 2 zeigt eine perspektivische Detailansicht einer ersten Ausgestaltung einer ersten Stützplatte 5 und einer zweiten Stützplatte 6, wie sie bei der Anordnung 28 aus Fig. 1 verwendet werden kann.

Die Rohröffnungen 11.1,11.2 der ersten Stützplatte 5 sind jeweils von einem ringförmigen Steg 14.1 umgeben, wobei die Stege 14.1 jeweils eine minimale Breite b haben, welche bei den Stegen 14.1 gleich groß ist. Die Rohröffnungen 12.1, 12.2 der zweiten Stützplatte 6 sind jeweils von einem ringförmigen Steg 14.2 umgeben, wobei die Stege 14.2 jeweils eine minimale Breite b haben, welche bei den Stegen 14.2 gleich groß ist.

Jedes der Rohre 4 der dritten Rohrgruppe 9 ist durch eine jeweilige Rohröffnung 12.2 der zweiten Stützplatte 6 geführt, welche kongruent zu der Rohröffnung 11.2 der ersten Stützplatte 5 liegt, durch welche das jeweilige Rohr 4 der dritten Rohrgruppe 9 geführt ist. Die Rohre 4 der ersten Rohrgruppe 7, der zweiten Rohrgruppe 8 und der dritten Rohrgruppe 9 sind nicht dargestellt.

Die Aussparungen 13.1 der ersten Stützplatte 5 weisen jeweils eine Sternform mit sechs Zacken auf und die Aussparungen 13.2 der zweiten Stützplatte 6 weisen jeweils eine Sternform mit sechs Zacken auf.

Jedes Rohr 4 der dritten Rohrgruppe 9 ist von jeweils drei Rohren 4 der ersten Rohrgruppe 7 umgeben und jedes Rohr 4 der dritten Rohrgruppe 9 ist von jeweils drei Rohren 4 der zweiten Rohrgruppe 8 umgeben.

Dementsprechend ist jede der Rohröffnungen 11.2 der ersten Stützplatte 5 von jeweils drei Rohröffnungen 11.1 der ersten Stützplatte 5 umgeben und jede der Rohröffnungen 12.2 der zweiten Stützplatte 6 ist von jeweils drei Rohröffnungen 12.1 der zweiten Stützplatte 6 umgeben.

Die erste Stützplatte 5 und die zweite Stützplatte 6 bilden zusammen das Stützelement 20 aus.

Die ringförmigen Stege 14.1 der ersten Stützplatte 5 sind über einen jeweiligen ersten Übergang 15.1 verbunden und die ringförmigen Stege 14.2 der zweiten Stützplatte 6 sind über einen jeweiligen zweiten Übergang 15.2 verbunden.

Der Vorteil ist, dass die Rohre 4 der ersten Rohrgruppe 7 und die Rohre 4 der zweiten Rohrgruppe 8 über die Rohre 4 der dritten Rohrgruppe 9 mechanisch gekoppelt werden. Dies hat eine erhöhte Stabilität des Rohrbündels 3 im Reaktorbehälter 2 zur Folge und bessere Dämpfungseigenschaften gegenüber Schwingungen der Rohre 4 im Betrieb. Weiterhin kann ein Knicken der Rohre beim Transport des Reaktorbehälters 2 vorteilhafter verhindert werden. Weiterhin wird die Hinderniswirkung durch diese Ausgestaltung der ersten Stützplatte 5 und der zweiten Stützplatte 6 minimiert.

### Bezugszeichenliste

- 1.1, 1.2: Vorrichtung
- 2: Reaktorbehälter
- 3: Rohrbündel
- 4: Rohre
- 5: erste Stützplatte
- 6: zweite Stützplatte
- 7: erste Rohrgruppe
- 8: zweite Rohrgruppe
- 9: dritte Rohrgruppe
- 10: Längsachse
- 11.1, 11.2: Rohröffnung der ersten Stützplatte
- 12.1, 12.2: Rohröffnung der zweiten Stützplatte
- 13.1,13.2: Aussparung
- 14.1, 14.2: Steg
- 15.1,15.2: Übergang
- 16: erstes Ende
- 17: zweites Ende
- 18: Verteiler
- 19: Sammler
- 20: Stützelement
- 21: gesättigter Dampf
- 22: Wasser leicht unter bzw. am Siedepunkt
- 23: Mantelraum
- 24: Synthesegaseinlass
- 25: vorgeheiztes Synthesegas
- 26: Gaseinlass
- 27: Produktgasauslass
- 28: Anordnung
- 29: Katalysator
- 30: Kondensator
- b: minimale Breite

## Patentansprüche

1. Vorrichtung (1.1;1.2) umfassend einen Reaktorbehälter (2), ein Rohrbündel (3) mit mehreren Rohren (4), eine erste Stützplatte (5) und eine zweite Stützplatte (6),
wobei das Rohrbündel (3) im Reaktorbehälter (2) angeordnet ist,
wobei das Rohrbündel (3) eine erste Rohrgruppe (7), eine zweite Rohrgruppe (8) und eine dritte Rohrgruppe (9) umfasst,
wobei die erste Stützplatte (5) und die zweite Stützplatte (6) quer zu einer Längsachse (10) des Reaktorbehälters (2) im Reaktorbehälter (2) angeordnet sind,
wobei jedes der Rohre (4) der ersten Rohrgruppe (7) durch eine jeweilige Rohröffnung (11.1) der ersten Stützplatte (5) geführt ist, und wobei die erste Stützplatte (5) mehrere Aussparungen (13.1) zum Fluidaustausch aufweist, wobei jedes der Rohre (4) der zweiten Rohrgruppe (8) durch eine jeweilige der Aussparungen (13.1) in der ersten Stützplatte (5) geführt ist,
wobei jedes der Rohre (4) der zweiten Rohrgruppe (8) durch eine jeweilige Rohröffnung (12.1) der zweiten Stützplatte (6) geführt ist, und wobei die zweite Stützplatte (6) mehrere Aussparungen (13.2) zum Fluidaustausch aufweist, wobei jedes der Rohre der ersten Rohrgruppe (7) durch eine jeweilige der Aussparungen (13.2) in der zweiten Stützplatte (6) geführt ist,
wobei jedes der Rohre (4) der dritten Rohrgruppe (9) durch eine jeweilige Rohröffnung (11.2) der ersten Stützplatte (5) geführt ist und durch die zweite Stützplatte (6) hindurchgeführt ist,
wobei die erste Stützplatte (5) die Rohre (4) der ersten Rohrgruppe (7) und der dritten Rohrgruppe (9) in den Rohröffnungen (11.1, 11.2) der ersten Stützplatte (5) quer zur Längsrichtung der Rohre (4) stützt und die zweite Stützplatte (6) die Rohre (4) der zweiten Rohrgruppe (8) in den Rohröffnungen (12.1) der zweiten Stützplatte (6) stützt.

2. Vorrichtung (1.1;1.2) nach Anspruch 1, wobei die Rohröffnungen (11.1,11.2) der ersten Stützplatte (5) jeweils von einem ringförmigen Steg (14.1) umgeben sind, wobei die Stege (14.1) jeweils eine minimale Breite (b) haben, welche bei zumindest einigen der Stege (14.1) gleich groß ist, und/oder
die Rohröffnungen (12.1) der zweiten Stützplatte (6) jeweils von einem ringförmigen Steg (14.2) umgeben sind, wobei die Stege (14.2) jeweils eine minimale Breite (b) haben, welche bei zumindest einigen der Stege (14.2) gleich groß ist.

3. Vorrichtung (1.1;1.2) nach einem der vorherigen Ansprüche, wobei jedes der Rohre (4) der dritten Rohrgruppe (9) durch eine jeweilige Rohröffnung (12.2) der zweiten Stützplatte (6) geführt ist, welche kongruent zu der Rohröffnung (11.2) der ersten Stützplatte (5) liegt, durch welche das jeweilige Rohr (4) der dritten Rohrgruppe (9) geführt ist.

4. Vorrichtung (1.1;1.2) nach einem der vorherigen Ansprüche, wobei die Aussparungen (13.1) der ersten Stützplatte (5) jeweils eine Sternform mit sechs Zacken aufweisen und/oder
die Aussparungen (13.2) der zweiten Stützplatte (6) jeweils eine Sternform mit sechs Zacken aufweisen.

5. Vorrichtung (1.1;1.2) nach einem der vorherigen Ansprüche, wobei jedes Rohr (4) der dritten Rohrgruppe (9) von jeweils drei Rohren (4) der ersten Rohrgruppe (7) umgeben ist und/oder jedes Rohr (4) der dritten Rohrgruppe (9) von jeweils drei Rohren (4) der zweiten Rohrgruppe (8) umgeben ist.

6. Vorrichtung (1.1;1.2) nach einem der vorherigen Ansprüche, wobei die erste Stützplatte (5) und die zweite Stützplatte (6) zusammen ein Stützelement (20) ausbilden.

7. Vorrichtung (1.1;1.2) nach Anspruch 6, wobei in dem Reaktorbehälter mehrere der Stützelemente (20) angeordnet sind, welche entlang der Längsachse (10) des Reaktorbehälters (2) zueinander versetzt sind.

8. Anordnung (28) zur Methanolsynthese umfassend eine Vorrichtung (1.1; 1.2) nach einem der Ansprüche 1 bis 8.

9. Verwendung einer Vorrichtung (1.11.2) nach einem der Ansprüche 1 bis 8, wobei Reaktionsedukte für die Methanolsynthese durch die Rohre (4) des Rohrbündels (3) geleitet werden und ein Kühlmedium außerhalb der Rohre (4) des Rohrbündels (3) durch den Reaktorbehälter (2) geleitet wird,
oder
wobei Reaktionsedukte für die Methanolsynthese außerhalb der Rohre (4) des Rohrbündels (3) durch den Reaktorbehälter (2) geleitet werden und ein Kühlmedium durch die Rohre (4) des Rohrbündels (3) geleitet wird.

10. Verfahren zur Methanolsynthese mit einer Vorrichtung (1.1; 1.2) nach einem der Ansprüche 1 bis 7, wobei Reaktionsedukte für die Methanolsynthese durch die Rohre (4) des Rohrbündels (3) geleitet werden und ein Kühlmedium außerhalb der Rohre (4) des Rohrbündels (3) durch den Reaktorbehälter (2) geleitet wird,
oder
wobei Reaktionsedukte für die Methanolsynthese außerhalb der Rohre (4) des Rohrbündels (3) durch den Reaktorbehälter (2) geleitet werden und ein Kühlmedium durch die Rohre (4) des Rohrbündels (3) geleitet wird.
